Europäisches Patentamt

(19) European Patent Office (11) Publication number: **0 046 609**

Office européen des brevets **B1**

(12) **EUROPEAN PATENT SPECIFICATION**

(45) Date of publication of patent specification: **21.11.84** (51) Int. Cl.³: **H 05 G 1/64, A 61 B 6/02**

(21) Application number: **81200839.9**

(22) Date of filing: **23.07.81**

54 **Radiography apparatus with a fan-shaped beam.**

(30) Priority: **21.08.80 NL 8004727**

(43) Date of publication of application:
**03.03.82 Bulletin 82/09**

(45) Publication of the grant of the patent:
**21.11.84 Bulletin 84/47**

(84) Designated Contracting States:
**DE FR GB NL**

(56) References cited:
**FR-A-1 174 835**
**FR-A-1 262 754**
**FR-A-2 059 074**
**FR-A-2 351 639**
**FR-A-2 352 532**
**FR-A-2 367 476**
**FR-A-2 368 058**
**FR-A-2 415 401**
**FR-A-2 431 236**
**US-A-3 925 660**
**US-A-4 179 100**

(73) Proprietor: **N.V. Philips' Gloeilampenfabrieken**
**Groenewoudseweg 1**
**NL-5621 BA Eindhoven (NL)**

(72) Inventor: **Haarman, Johan Willem**
**c/o INT. OCTROOIBUREAU B.V. Prof. Holstlaan 6**
**NL-5656 AA Eindhoven (NL)**

(74) Representative: **Scheele, Edial François et al**
**INTERNATIONAAL OCTROOIBUREAU B.V. Prof.**
**Holstlaan 6**
**NL-5656 AA Eindhoven (NL)**

Courier Press, Leamington Spa, England.

# Description

The invention relates to radiography apparatus, comprising a radiation source provided with a slit-shaped collimator, for generating a fan-shaped radiation beam, a radiation detection system including an image intensifier tube, a television camera tube and a non-spherical optical system for forming a rectangular image of a strip-shaped scintillation exit surface, an object carrier and a scanning mechanism for scanning an object under examination with a fan-shaped radiation beam generated by the radiation source.

Radiography apparatus of this kind are known from US 4,236,080. Apparatus described therein are equipped to spread a line image generated by an imaging system working with a fan beam over a rectangular part of the target of a television pick-up tube. For each subsequent layer image a new rectangular target image is produced. This method of measuring is rather time consuming but indeed the apparatus works with a relatively small radiation dosis for the object to be examined. In order to maintain this low dosis particularly for medical applications it is desirable that noise generated in the entire detection system should be so low that the noise present in the detected radiation will predominate in the noise present in the image, because then the situation is reached where the dose applied to the patient is minimal. It is also desirable that a total exposure of an object can be made within a comparatively short period of time without high loading of the source being necessary.

The invention has for its object to satisfy this requirement; to this end, radiography apparatus of the kind set forth is characterized in that the scanning mechanism causes an oscillatory movement of the detection system about a generating point for radiation from the source, in a direction transverse with respect to the fan direction of the fan-shaped radiation beam.

The source/detector system in an apparatus in accordance with the invention is arranged to be rotatable about the generating point for radiation from the source, so that a field of, for example, 40 × 35 cm² is scanned by an oscillatory movement about said point in a direction transverse with respect to the fan direction of the beam. All objects which are of interest from a medical point of view, such as organs etc., can thus be imaged in a single measurement process.

Cross-talk between image elements can be greatly influenced by utilizing a layer containing CsI as the scintillation material and by subjecting it to the process of forming discontinuities in the layer in the form of cracks, as described in GB 1,380,186. An adapted method can be used for forming a scintillator which exhibits some thirty optically separated lines in a longitudinal direction.

In a further preferred embodiment, the source can be controlled at a frequency of, for example, four pulses per second, each having a duration of 5 ms, so that an area of 35 × 40 cm² can be scanned using a translatory displacement of, for example, 4 cm per second, with 40 pulses, to provide 40 images of, for example, 30 lines each.

In a further embodiment, the television camera tube is adapted to scan a field without interlace in, for example, 1250 lines which are oriented transversely to the longitudinal direction of the image of the detector exit surface, so that the signal processing speed of the detection system is made to correspond to, for example, that of a magnetic disc memory.

In a further preferred embodiment, the scintillator and the brightness intensifier are both replaced by an X-ray image intensifier tube, for example, a 14″ (35.56 cms) tube such as described in British Patent Application No. 2,006,518.

In this embodiment, there may be arranged between the X-ray intensifier tube and the camera tube, an optical element by means of which strip-shaped images moving across the entrance face of the X-ray intensifier tube can be projected onto a fixed location on the target of the camera tube.

In a further preferred embodiment yet, the apparatus has a plurality of source positions which are situated, for example, along a line parallel to the longitudinal direction of the detector, so that tomosynthesis can be performed on the measurement signals, with the result that images of different slices can be formed in accordance with the method described in GB 1,332,280.

A further preferred embodiment is adapted to measure radiation of several, for example, two, different wavelengths so that, for example, subtraction images can be formed or discrimination can take place in relation to the density of given molecules or in relation to electron density and a direct relationship can be established between diagnosis and radiation therapy.

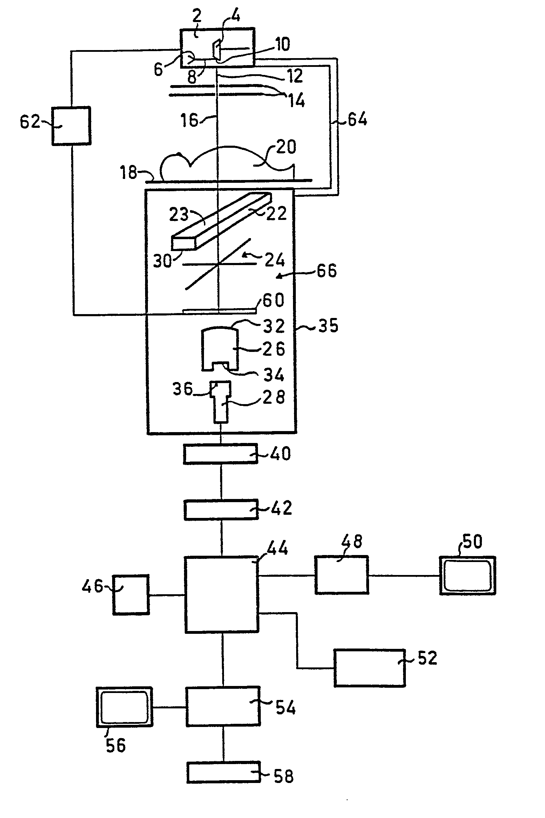

Some preferred embodiments in accordance with the invention will be described in detail hereinafter with reference to the accompanying drawing. The drawing diagrammatically shows radiography apparatus in accordance with the invention.

Radiology apparatus as shown in the drawing comprises an X-ray source 2 with an anode 4 and a cathode 6. An electron beam 8 emitted by the cathode is incident on the anode, in this case constructed as a rotary anode, at a target spot 10 where it generates an X-ray beam 12 which has, with a suitable approximation, the target spot 10 as its generating point. The beam 12 passes through a collimator 14 which is in this case shown as two diaphragms in succession. The collimator forms a fan-shaped, flat beam 16 for irradiating a patient 20 arranged on an object carrier 18. For the detection of transmitted radiation, the apparatus comprises a detection system 66 with a strip of scintillation material 22, a non-spherical optical lens system 24, a light intensifier 26 and a television camera tube 28. The lens system 24, being in principle a cylindrical lens, forms an at least approximately square image of an exit surface 30 of the scintillator 22, on an entrance screen 32 of the light intensifier. The light intensifier forms an intensified image of the entrance image on an exit screen 34 thereof. This image is transferred, by means of a lens optical system or a fibre optical plate; to an entrance screen 36 of the television camera tube 28. The television camera tube is preferably a "frog's head plumbicon" (45 QX) which is marketed by N. V. Philips and which is suitable for forming images with a resolution of up to approximately 2000 lines. For signal processing, the apparatus comprises a video amplifier 40, an analog-to-digital converter 42, and a central computer 44, for example, a Philips "P854". Also connected to the central computer are: a preferably digital optical recording system 46 for the storage of information, a signal converter 48 with a monitor 50 for display, a magnetic disc recording system 52, a signal converter 54 with a high-resolution monitor 56, and a device 58 for forming hard copies of the images. A sensor 60 controlling an electrical generator 62 for activating the X-ray source, is arranged in the beam path behind the patient. A coupling 64, for example, a mechanical coupling, ensures that a fixed geometry of the source 2 and the detector system 66 is maintained with respect to one another during scanning; this function can alternatively be performed by an electronic circuit. The displacement assembly is coupled to the generator 62 in order to ensure a suitable synchronization of the operations of scanning and source activation. The collimator 14 forms a fan-shaped X-ray beam 16, the transverse dimensions of which corresponds, after passing through an object under examination, to an active entrance surface 23 of the detector. This surface has, for example, a length of from 35 to 40 cm and a width of 1 cm. For the scintillation material, use can be made of CsI which has to be shielded in view of the hygroscopic nature thereof, from the ambient atmosphere. The strip of scintillation material is made of optically mutually isolated elements in a preferred embodiment. A suitable method of forming these elements is the process of generating discontinuities in the form of cracks, in a phosphor layer which is provided, for example, on a structured carrier for this purpose. The carrier may be present at the entrance side 23 or at the exit side 30. In the former case it should be suitably transparent to X-rays and, for example, a thin, structured foil of titanium can be used very suitably. When the carrier is situated at the exit surface, it should be suitably transparent to scintillation light and may consist of, for example, quartz glass. The carrier may extend further to form a housing for the entire scintillator, so that shielding against atmospheric deterioration is ensured. The process of crack formation divides the various scintillator elements into pillars having, for example, a dimension which at the most equals 0,25 mm, measured in the plane of the layer. In order to reduce cross-talk, the gaps formed by the process of crack formation may be filled with a substance which strongly reflects or absorbs the scintillation light. The beam of scintillation light is imaged on an entrance surface 32 of the light intensifier 26; if desired, the light beam may be deflected by means of a mirror, so that the light intensifier and the camera tube are differently oriented and a smaller overall depth would suffice for the detection system. On the entrance screen 32, the lens system 24 forms a square image of, for example, approximately 8 cm × 8 cm of the exit surface 30 of the scintillator which has transverse dimensions of, for example, 35 cm × 1 cm. When use is made of, for example, a 4" inch light intensifier, the function of the non-spherical lens system can be performed by the optical deflection system which may then be shaped as a concave cylindrical mirror. The light intensifier forms an intensified exit image from the entrance image in known manner, said exit image being projected onto the entrance screen 36 of the camera tube 28. This image is scanned in, for example, 1250 lines without the interlace which is customarily used in television; for which purpose the camera tube must have a high resolution. A video signal thus recovered is applied to a video amplifier 40. The system formed by the scintillator, the lens system, the light intensifier and the camera tube is accommodated, for example, in a light-tight housing 35; this hous-ing takes part in the same displacement as the source/detector system which together scan the patient as a single unit. For this displacement, a pivot may be chosen to coincide with the generating point 10 of the X-ray beam. According to this method of scanning, the source/detector system with the coupling 64 is tilted about the tilting point 10 in a plane which coincides with the plane of the drawing. Each time adjacent sectional regions of the patient, which are not entirely parallel to one another in this case, are then scanned. Images of mutually parallel sections are formed during a scan during which the source and detector together perform a translatory displacement. The source is then pulsed with, for example, 4 pulses per second each of 5 ms duration. Each pulse is picked up by the camera tube and is recorded. The direction of the longitudinal dimension of the detector image on the entrance screen of the camera tube then extends transversely with respect to the line scan direction of the electron beam of the camera tube. The camera tube screen is erased during the pulses. During this scan, the detector is displaced at a speed of, for example, 4 cm per second over a total distance of 40 cm, i.e. there are 40 pulses and hence 40 sectional irradiations. For each exposure, a field of 35 × 40 cm², divided into 1250 × 1200 image elements, is thus recorded, *i.e.* 1250 due to the number of lines in the line scan and 1200 in that 30 paths are scanned 40 times.

In a preferred embodiment in which the scintillator material incorporated in the light intensifier forms an X-ray image intensifier tube, the non-spherical optical system is situated between this tube and the camera tube. Because the dimensions of the image in the X-ray image intensifier tube would then be substantially reduced, the non-spherical optical system may have a simpler construction or a higher optical quality.

Using an optical element, for example, a rotatable mirror or a prism arranged at the exit side of the X-ray intensifier tube, in this case preferably a 14″ tube as described in US 4,213,055, an exit image of a strip-shaped image moving across the entrance screen thereof can be continuously projected onto a desired, for example, fixed location on the entrance screen of the camera tube. Motional unsharpness which would be produced by movement of the beam within an X-ray source pulse duration can thus be reduced by way of an adapted, oppositely directed movement of the exit image by means of the optical element. When this compensation is applied, the pulse duration of the X-ray source may be longer, so that limitation of the beam intensity by excessive local heating of the anode of the X-ray source can be prevented. An optical element which is suitable for this embodiment is described in US 4,236,080, so it need not be elaborated herein.

If the radiation source is formed by the patient himself, for example, due to a radioactive tracer introduced therein, the slit-shaped collimator is arranged between the patient and the scintillator. The scintillator is then adapted to the nature of the radiation to be measured and will usually be much thicker to ensure adequate absorption of the comparatively hard radiation.

For a permissible source load, a favourable compromise can be found between the measurement rate and the quality of the image formation by selection of the number of separately readable paths which are operative and read during each radiation pulse. The limitation in the dynamic range of the camera tube is always compensated for by forming a substantially square image of the path pattern on the target and, should this still be insufficient, by using a slow scan mode in the camera tube.

## Claims

1. Radiography apparatus, comprising a radiation source (2) provided with a slit-shaped collimator (14) for generating a fan-shaped radiation beam (16), a radiation detection system (66) including an image intensifier tube (26), a television camera tube (28) and a non-spherical optical system (24) for forming an at least substantially square image of a strip-shaped scintillator exit surface (30), an object carrier (18) and a scanning mechanism (35) for scanning an object (20) under examination with the fan-shaped radiation beam generated by the radiation source, characterized in that the scanning mechanism (2, 64, 35) causes an oscillatory movement of the detection system about a generating point (10) for radiation from the source, in a direction transverse with respect to the fan direction of the fan-shaped radiation beam.

2. Radiography apparatus as claimed in Claim 1, charaterized in that behind the entrance screen (32) of the image intensifier tube (26) there is arranged an optical element, for the dynamic movement of the image in a direction which coincides with the scanning direction of the radiation beam.

3. Radiography apparatus as claimed in Claim 1 or 2, characterized in that the radiation source is formed by an X-ray tube with a generator (62) for pulsed operation of the source.

4. Radiography apparatus as claimed in Claim 2, characterized in that the generating point of the source can be switched between several positions which are situated along a line parallel to the longitudinal direction of the detector.

5. Radiography apparatus as claimed in Claim 3 or 4, characterized in that the generator for the X-ray tube is adapted to generate X-rays of different wavelengths.

## Revendications

1. Appareil de radiographie, comportant une source de rayonnement (2) muni d'un collimateur en forme de fente (14), servant à engendrer un faisceau de rayons en forme d'éventail (16), un système de détection de rayonnement (66) comprenant un tube intensificateur d'image (26), un tube de prise de vues de télévision (28) et un système optique non sphérique (24) servant à former une image au moins pratiquement carrée d'une surface de sortie de scintillation en forme de bande, un porteur d'objet (18) et un mécanisme de balayage (35) servant à balayer un objet (20) à examiner avec un faisceau de rayons en forme d'éventail engendré par la source de rayonnement, caractérisé en ce que le mécanisme de balayage (2, 64, 35) provoque un mouvement d'oscillation du système de détection par rapport à un point (10) servant à la formation du rayonnement de la source, dans une direction transversale par rapport à la direction d'éventail du faisceau de rayonnement en forme d'éventail.

2. Appareil de radiographie selon la revendication 1, caractérisé en ce que derrière l'écran d'entrée (32) du tube intensificateur d'image (26) est prévu un élément optique pour le déplacement dynamique de l'image dans une direction, qui coïncide avec la direction de balayage d'un faisceau de rayonnement.

3. Appareil de radiographie selon la revendication 1 ou 2, caractérisé en ce que la source de rayonnement est formée par un tube de rayons X présentant un générateur 62 pour le fonctionnement pulsé de la source.

4. Appareil de radiographie selon la revendication 2, caractérisé en ce que le point de formation de la source peut être commuté entre plusieurs positions qui sont situées suivant une ligne parallèle à la direction longitudinale du détecteur.

5. Appareil de radiographie selon la revendication 3 ou 4, caractérisé en ce que le générateur pour le tube à rayons X convient à former des rayons X de plusieurs longueurs d'onde.

**Patentansprüche**

1. Röntgengerät mit einer Strahlungsquelle (2) mit einem schlitzförmigen Kollimator (14) zum Erzeugen eines fächerförmigen Strahlenbündels (16), mit einem Strahlungsdetektorsystem (66) mit einer Bildverstärkerröhre (26), einer Fernsehkameraröhre (28) und einem asphärischen optischen System (24) zur Bildung eines zumindest im wesentlichen viereckigen Bildes einer streifenförmigen Szintillatorausgangsfläche (30), mit einem Objektträger (18) und einer Abtastanordnung (35) zum Abtasten eines zu untersuchenden Objekts (20) mit dem von der Strahlungsquelle erzeugten, fächerförmigen Strahlenbündel, dadurch gekennzeichnet, dass die Abtastanordnung (2, 64, 35) eine Schwingungsbewegung des Detektorsystems um einen Erzeugungspunkt (10) der Strahlung aus der Quelle in einer Senkrecht zur Fächerrichtung des fächerförmigen Strahlenbündels verlaufenden Richtung bewirkt.

2. Röntgengerät nach Anspruch 1 dadurch gekennzeichnet, dass hinter dem Eintrittsschirm (32) der Bildverstärkerröhre (26) ein optisches Element für die dynamische Bewegung des Bildes in einer Richtung angeordnet ist, die mit der Abtastrichtung des Strahlenbündels zusammenfällt.

3. Röntgengerät nach Anspruch 1 oder 2, dadurch gekennzeichnet, dass die Strahlungsquelle durch eine Röntgenröhre mit einem Generator (62) für einen Pulsbetrieb der Quelle gebildet wird.

4. Röntgengerät nach Anspruch 2, dadurch gekennzeichnet, dass der Erzeugungspunkt der Quelle zwischen mehreren Stellungen schaltbar ist, die sich auf einer parallel zur Längsrichtung des Detektors verlaufenden Linie befinden.

5. Röntgengerät nach Anspruch 3 oder 4, dadurch gekennzeichnet, dass der Generator für die Röntgenröhre zum Erzeugen von Röntgenstrahlen mit verschiedenen Wellenlängen ausgelegt ist.

0 046 609

1